# EUROPÄISCHE PATENTSCHRIFT

(11) **EP 2 187 209 B1**
(45) Veröffentlichungstag und Bekanntmachung des Hinweises auf die Patenterteilung: **28.03.2018**
(21) Anmeldenummer: 08450180.8
(22) Anmeldetag: 12.11.2008
(51) Int. Cl.: G01N 33/49, G01N 21/03, G01N 21/05

(54) **Hämolysator**
Hemolyser
Hémolysateur

(43) Veröffentlichungstag der Anmeldung: 19.05.2010
(73) Patentinhaber: Roche Diagnostics GmbH, 68305 Mannheim (DE); F. Hoffmann-La Roche AG, 4070 Basel (CH)
(72) Erfinder: Huemer, Herfried, 8330 Feldbach (AT)
(74) Vertreter: Margotti, Herwig Franz

(56) Entgegenhaltungen:
- EP-A- 1 764 608
- WO-A1-2006/119932
- DE-A1- 19 930 585
- DE-T2- 60 011 253
- GB-A- 2 403 729
- US-A- 3 972 614
- US-A- 4 983 523
- US-A- 5 869 767
- US-A- 6 100 084
- US-B2- 6 739 531
- TAYLOR M T ET AL: "Disrupting bacterial spores and cells using ultrasound applied through a solid interface" 2ND ANNUAL INTERNATIONAL IEEE-EMBS SPECIAL TOPIC CONFERENCE ON MICROTECHNOLOGIES IN MEDICINE AND BIOLOGY. CONFERENCE PROCEEDINGS (CAT. NO. 02EX578); IEEE, PISCATAWAY, NJ, USA, 2002, Seiten 551-555, XP002529966 ISBN: 0-7803-7480-0

## Beschreibung

Die Erfindung betrifft einen Hämolysator mit Multilayer-Aktuatoren und mit einer austauschbaren Küvette zur Aufnahme einer Probe, insbesondere einer Blutprobe, im Probenraum des Hämolysators. Die Erfindung betrifft auch einen spektroskopischen Analysator mit einem solchen Hämolysator mit austauschbarer Küvette im Probenraum. Die Erfindung betrifft weiters ein Verfahren zum Betreiben eines mit piezoelektrischen Multilayer-Aktuatoren ausgestatteten Hämolysators, in dessen Probenraum eine austauschbare Küvette eingesetzt wird. Ein eingangs genannter Hämolysator findet Verwendung in einem spektroskopischen Analysator, insbesondere zur spektroskopischen Bestimmung von Hämoglobinderivaten, z.B. O₂Hb, HHb, COHb, MetHb und davon abgeleiteter Größen (Oxymetrie bzw. Cooxymetrie). Der Hämolysator ist aber auch zum Einsatz in kombinierten spektroskopischchemischen Analysatoren geeignet. Die genannten Analysatoren dienen unter anderem zur dezentralen Bestimmung der Blutgase (O₂, CO₂, pH), der Elektrolyte (K⁺, Na⁺, Ca⁺⁺, Cl⁻), der Metabolite (Glukose und Laktat), des Hämatokrits, der Hämoglobinparameter (tHb, SO₂, etc.) und Bilirubin in Vollblutproben. Dabei werden charakteristische photometrische Absorptionseigenschaften dieser Substanzen genutzt und die Messwerte über einen mathematischen Algorithmus ausgewertet.
Um die geforderte Messgenauigkeit erreichen zu können, ist eine Hämolyse des Vollblutes vor der optischen Messung notwendig, die mit Hilfe des Hämolysators durchgeführt wird. Dabei werden die Blutzellen zumeist durch Ultraschallenergie zerstört, um eine Messung ohne störende Lichtstreuungseffekte durchführen zu können.

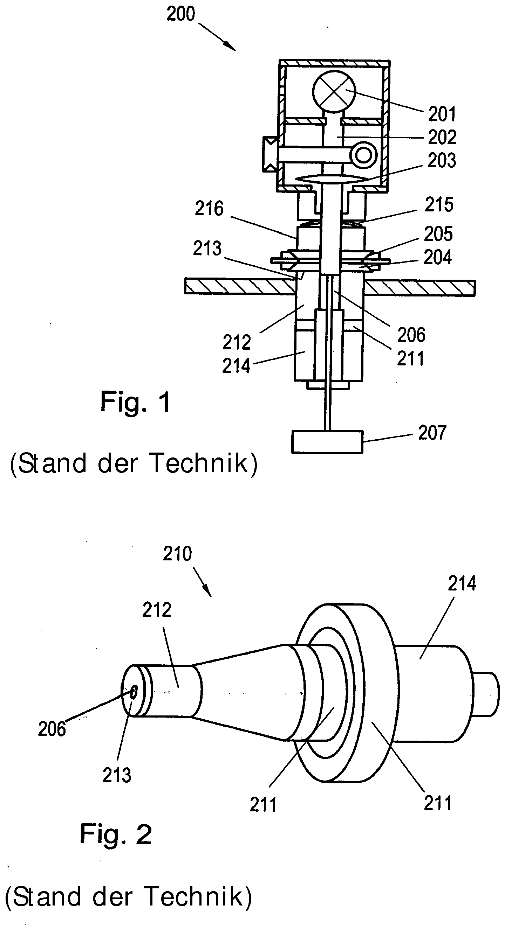

In Fig. 1 ist ein Prinzipschaubild eines aus dem Stand der Technik bekannten Oxymetermoduls 200 eines spektroskopischen Analysators dargestellt. Dieses Oxymetermodul umfasst eine Messlichtquelle 201, die einen Lichtstrahl 202 erzeugt, der durch eine Linse 203 gebündelt und auf eine optische Messkammer 204 gerichtet wird.

Diese auch als "Küvette" bezeichnete optische Messkammer 204 weist transparente Gehäusewände auf, durch die der Lichtstrahl 202 hindurchtreten kann. In der Messkammer 204 befindet sich eine Probe 205, insbesondere eine Vollblutprobe, mit charakteristischen photometrischen Absorptionseigenschaften, die die spektrale Zusammensetzung des durch die Probe hindurchtretenden Lichtstrahls 202 verändern. Der Lichtstrahl 202 wird nach dem Verlassen der Messkammer 204 in einen Lichtwellenleiter 206 eingeleitet und zu einem spektroskopischen Sensor 207 geführt. Die spektroskopische Analyse der Probe 205 setzt eine Hämolyse voraus, bei der in einer Vollblutprobe die Blutzellen durch Ultraschall zerstört werden, so dass die Probe in eine den Lichtstrahl 202 im Wesentlichen nicht streuende Flüssigkeit transformiert wird. Die Hämolyse wird mittels eines Hämolysators 210 durchgeführt, der als Ultraschall-Transducer ausgeführt ist und piezokeramische Elemente 211 umfasst, die durch Anregung mit elektrischen Wechselstromsignalen aufgrund des umgekehrten Piezoeffekts (d.h. des physikalischen Phänomens, bei dem durch Anlegen von elektrischen Signalen an ein Piezoelement mechanische Verformungen hervorgerufen werden) mechanische Schwingungen erzeugen, die auf einen Resonator 212 übertragen und verstärkt werden (siehe auch Fig. 2). Der Resonator 212 überträgt seinerseits über eine an seiner Stirnseite ausgebildete Ankopplungsfläche 213 die mechanischen Schwingungen auf eine Gehäusewand der Messkammer 204, wodurch sich die Schwingungen in die Probe 205 fortpflanzen und darin die Blutzellen durch Kavitationseffekte platzen lassen. Der Hämolysator 210 besitzt weiters eine Gegenmasse 214, die an der dem Resonator 212 abgewandten Seite der piezokeramischen Elemente 211 angeordnet ist. Damit die Messkammer 204 durch die mechanischen Schwingungen nicht zerstört wird und um die geeignete Fortpflanzung der mechanischen Schwingungen in die Probe 205 sicherzustellen, muss sie federnd gelagert sein. Dies erfolgt durch eine Federscheibe 215, die einen Amboss 216 gegen jene Seite der Messkammer 204 vorspannt, die dem Hämolysator 210 gegenüberliegt.
Gemäß dem Stand der Technik wurden Hämolysatoren 210 des Ultraschall-Transducer Typs bisher als Resonanzschwinger konfiguriert, die durch ein elektrisches Sinussignal mit der dem Hämolysator eigenen Resonanzfrequenz zur Schwingung angeregt werden. Der in Fig. 2 dargestellte Ultraschall-Transducer-Hämolysator 210 ist ein solcher auf dem Resonanzschwingerprinzip basierender Hämolysator 210 nach dem Stand der Technik.
Spektroskopische Analysatoren mit Hämolysatoren nach dem Resonanzschwingerprinzip sind beispielsweise aus der US 3,972,614 bekannt. Weitere Hämolysatoren sind aus der WO 2006/119932 und der US 6,739,531 bekannt. Nachteilig an den bekannten Hämolysatoren vom Resonanzschwinger-Ultraschall-Transducer Typ ist ihre beträchtliche Baulänge, die so bemessen werden muss, dass eine maximale Schwingungsamplitude an der Probenposition erzielt wird. Der Resonanzschwinger-Ultraschall-Transducer ist ein λ/2-Schwinger, der eine Baulänge von einigen cm (typischerweise ca. 10 cm) erfordert. Dies führt zu voluminösen Analysatoren.
Weiters muss der Resonanzschwinger aufgrund seines Wirkungsprinzips der Anregung bei Resonanzfrequenz eine sehr hohe Güte aufweisen, die dazu führt, dass er nur in einem äußerst schmalen Frequenzbereich betrieben werden kann. Dies schränkt erstens die Steuermöglichkeiten des Hämolysevorgangs ein, denn in Abhängigkeit von der Ultraschallfrequenz entstehen in der Blutprobe Kavitationsblasen verschiedener Größe und Dichte. Zweitens besteht im Fall von austauschbaren Messkammern (Küvetten), in denen die Hämolyse durchgeführt werden soll, das Problem unterschiedlicher und über die Lebensdauer veränderlicher Material- und Schwingungseigenschaften. Diese führt bei Übertragung von mechanischen Schwingungen durch einen - prinzipbedingt auf eine fixe Frequenz eingestellten - Resonanzschwinger-Ultraschall-Transducer dazu, dass Fehlabstimmungen im Frequenzverhalten auftreten können, die zu unzureichender Hämolyse der Blutprobe in der Küvette führen. Drittens wäre es auch vorteilhaft, Qualitätsüberprüfungen und Systemtests im Betrieb durchführen zu können, wofür Resonanzschwinger mit ihrem sehr schmalen nutzbaren Frequenzbereich aber ebenfalls untauglich sind.
Schließlich ist auch der relativ hohe Energieverbrauch bekannter Resonanzschwinger-Ultraschall-Transducer problematisch.
Zusammengefasst wäre es daher wünschenswert einen Hämolysator für ein Oxymetermodul eines spektroskopischen Analysators bereitstellen zu können, der klein und leise ist, einen geringen Energieverbrauch von <= 15 W Dauerleistung aufweist, an dem interne Qualitätskontrollen durchführbar sind, der es ermöglicht, dass auch bei einem Küvettentausch, der die Schwingungseigenschaften des Systems verändert, das System betreibbar bleibt, und der eine vollständige Hämolyse der Blutprobe durchführt.
Die Erfindung löst die gestellten Aufgaben durch das Bereitstellen eines Hämolysators mit den kennzeichnenden Merkmalen des Anspruchs 7. Vorteilhafte Ausgestaltungen der Erfindung sind in den Unteransprüchen dargelegt.
Der erfindungsgemäße Hämolysator umfasst eine Sonotrodenplatte und darauf wirkende Schwingungserzeugungselemente, wobei die Schwingungserzeugungselemente von einem elektrischen AC-Signalgenerator in mechanische Schwingungen versetzbar sind, und einen Probenraum, auf den die Sonotrodenplatte mechanische Schwingungen überträgt. Erfindungsgemäß sind die Schwingungserzeugungselemente in einem breiten Frequenzband von 20 - 50 kHz durchstimmbar zu mechanischen Schwingungen anregbar. Der Begriff "in einem breiten Frequenzband anregbar" ist so zu verstehen, dass einerseits aufgrund ihrer Signalform breitbandige Schwingungen erzeugbar sind, andererseits auch innerhalb des breiten Frequenzbandes schmalbandige Schwingungen, z.B. Sinusschwingungen mit diskreter Frequenz erzeugt werden können. In einer Fortbildung der Erfindung ist vorgesehen, dass (schmalbandige) Schwingungen durch das breitbandige Frequenzband hindurch durchgestimmt werden.
Geeignete Frequenzbereiche im Sinne der vorliegenden Erfindung sind allgemein alle Frequenzbereiche, in welchen eine ultraschallbedingte Hämolyse stattfinden kann. Insbesondere sind dies Frequenzbereiche, welchen ultraschallbedingt eine Bildung von Kavitationsblasen im Medium bewirken können, welche ihrerseits eine Zerstörung der im Medium enthaltenen Zellen bewirken können. Diese Frequenzbereiche liegen im Bereich von 20 -50 kHz. Tiefere Frequenzbereiche haben den Nachteil, daß es hier aufgrund der Überschneidung mit dem hörbaren Frequenzspektrum zu wahrnehmbaren Geräuscherscheinungen kommen kann. Höhere Frequenzbereiche haben den Nachteil, daß es hier zu hohen Energieverlusten bzw. geringeren Wirkungsgraden, beispielsweise durch Erwärmung des Hämolysators, kommen kann.
Die Kernidee der vorliegenden Erfindung ist somit, einen Hämolysator bereitzustellen, der nicht auf dem Resonanzschwingerprinzip (λ/2-Resonator, der von Piezoelementen angeregt wird) beruht, welcher prinzipbedingt sehr schmalbandige und nicht durchstimmbare mechanische Schwingungen erzeugt, sondern stattdessen einen Ultraschall-Transducer umfasst, der mechanische Schwingungserzeugungselemente aufweist, die in einem breiten Frequenzband durchstimmbar durch elektrische Wechselsignale zu mechanischen Schwingungen angeregt werden können. Mithilfe solcher breitbandiger mechanischer Schwingungserzeugungselemente ist es möglich, deren Anregung in Hubamplitude und Hubfrequenz so zu regeln, dass der Hämolysator betreibbbar bleibt, auch wenn sich die internen Schwingungseigenschaften des Systems (beispielsweise nach einem Tausch von Baugruppen, wie Küvetten) verändern. Dies ist besonders wichtig für ein neuartiges Konzept, für das die Erfindung besonders geeignet ist, bei welchem Konzept die Küvette ein Verbrauchsmaterial darstellt oder Bestandteil eines Verbrauchsgutes ist, das nachfolgend auch als "Consumable" bezeichnet wird, wobei es ein Kennzeichen eines solchen Consumable ist, dass es turnusmäßig ausgetauscht wird. Man muss beim Tausch solcher Consumables in Kauf nehmen, dass aufgrund unvermeidlicher Schwankungen von Materialeigenschaften zwischen verschiedenen Chargen von Consumables die mechanischen und Materialeigenschaften der darin integrierten Austausch-Küvetten nicht immer exakt dieselben sind.

Idealerweise sollen Analysatoren mit dem erfindungsgemäßen Hämolysator auch für den "untrainierten" Benutzer einfach und intuitiv zu bedienen sein. Eine weitere wichtige Anforderung an ein solches Gerät ist, dass es aus Sicht des Anwenders "beinahe wartungsfrei" zu bedienen ist. Unter "beinahe wartungsfrei" wird allgemein verstanden, dass auch der (technisch) ungeschulte Anwender zum laufenden Betrieb lediglich in Form von Kassetten und/oder Modulen vorliegende Verbrauchsmaterialien tauscht. Alle Verbrauchsmaterialien (Consumables) sollen durch einfache intuitive Handgriffe vom Anwender turnusmäßig tauschbar sein, beispielsweise nach Ablauf einer bestimmten Einsatzdauer oder nach Erreichen einer bestimmten Anzahl von Messungen oder nach Verbrauch der darin bereitgestellten Betriebsmittel.

In Systemen nach dem Stand der Technik, wie z.B. im US Patent 3,72,614 beschrieben, wurde die optische Messkammer (Küvette) als integriertes Bestandteil des Oxymeters ausgeführt, welches permanent im Gerät verbleibt. Der in US 3,972,614 beschriebene Analysator zur spektroskopischen Bestimmung von Parametern in Blutproben, wie z.B. Hämoglobin, umfasst auch Mittel zur Ultraschall-Hämolyse der Blutprobe, d.h. der Zerstörung der roten Blutkörperchen, um die Blutprobe möglichst streukörperfrei zu machen. Erst dadurch wird eine genaue spektroskopische Analyse der Probe möglich.

Nachteilig wirkt sich bei dem bekannten System jedoch das hohe Verstopfungsrisiko während der geplanten Einsatzdauer aus, wobei insbesondere die fluidischen Koppelstellen sowie eine geringe Schichtdicke des Probenkanals Problempunkte darstellen. Falls bei diesen Oxymetersystemen Verschmutzungen oder Verstopfungen im Bereich der Küvette auftauchen, können diese oft nur durch einen aufwendigen Tausch der Küvette behoben werden. Hierzu ist meist eine entsprechende Schulung oder die Anforderung eines Servicetechnikers nötig, so dass häufig längere unplanmäßige Ausfallzeiten des Analysators die Folge sind. Weiterhin bedarf der manuelle Austausch einer Küvette in solchen Systemen oft nachfolgender manueller Justage- und Kalibrierungsschritte, um wieder reproduzierbare Messergebnisse zu erhalten.

Wie bereits oben erklärt, ist der erfindungsgemäße Hämolysator aufgrund seiner Vielseitigkeit in der elektrischen Ansteuerung, seiner Durchstimmbarkeit in Hubamplitude und Hubfrequenz hervorragend für Analysator-Systeme geeignet, bei denen die Hämolyse direkt in austauschbaren Küvetten durchgeführt wird, insbesondere für Analysator-Systeme, bei denen Küvetten verwendet werden, welche auch für den "untrainierten" Benutzer einfach und intuitiv zu wechseln sind, beispielsweise, weil die Küvetten Teil eines turnusmäßig austauschbaren Consumables sind. Der erfindungsgemäße Hämolysator ist dadurch gekennzeichnet, dass in den Probenraum eine austauschbare Küvette eingesetzt ist, die einen Probenkanal zur Aufnahme einer Blutprobe aufweist, der durch zumindest eine schwingungsübertragende Wand begrenzt wird, wobei im eingesetzten Zustand die schwingungsübertragende Wand der Küvette gegen die Sonotrodenplatte anliegt.

Die Erfindung betrifft eine Kombination des erfindungsgemäßen Hämolysators mit eingesetzter Küvette, wobei die Küvette zumindest ein Dichtungselement und zwei transparente Elemente umfasst, wobei die beiden transparenten Elementen im Abstand voneinander angeordnet sind und zwei einander gegenüberliegende Begrenzungsflächen eines Probenkanals definieren und das zumindest eine Dichtungselement Seitenwände des Probenkanals definiert, wodurch der Probenkanal als ein in Längsrichtung geschlossener Kanal mit einer Einlassöffnung und einer Auslassöffnung ausgebildet ist. Es ist zumindest ein Abstandshalter vorgesehen, der die transparenten Elemente im Abstand voneinander hält. Zumindest eines der beiden transparenten Elemente weist einen Absatz auf, der sich in Richtung zum anderen transparenten Element erstreckt und eine Begrenzungsfläche des Probenkanals bildet, so dass die Höhe des Probenkanals geringer ist als die Höhe des zumindest einen Abstandshalters.
Durch das Vorsehen des Absatzes an zumindest einem der beiden transparenten Elemente kann eine wohldefinierte Probenkanalhöhe erzielt werden, die kleiner ist als die Höhe des Abstandshalters, z.B. 0,1 oder 1 mm hoch. Dabei sind aber sowohl die beiden transparenten Elemente als auch der Abstandshalter als solide Elemente mit ausreichender Dicke ausgebildet, um die erforderliche hervorragende mechanische Stabilität und Maßhaltigkeit aufweisen zu können und um für Ultraschall-Hämolyse geeignet zu sein.
Damit der Abstandshalter auch bei Ultraschall-Applikationen über seine Lebensdauer zufriedenstellende Maßhaltigkeit beibehält ist es bevorzugt, ihn aus einem spritzgussfähigen Kunststoff mit hohen E-Modulwerten von vorzugsweise mehr als 2500 MPa, noch bevorzugter mehr als 5000 MPa, zu fertigen.
Bei einer Ausführungsform der Küvette mit ausgezeichneter mechanischer Festigkeit und Maßhaltigkeit sind die transparenten Elemente mit dem Abstandshalter mittels eines formbeständigen Klebstoffs verklebt, der eine definierte Schichtdicke aufweist. Durch Justieren der Position der transparenten Elemente vor dem Aushärten des Klebers ist eine exakte Probenkanalhöhe einstellbar.

In einer alternativen Ausführungsform sind die transparenten Elemente der Küvette mit dem Abstandshalter verpresst, zusammengesteckt oder mittels Klammern verbunden. Auch bei dieser Ausführungsform wird eine hohe Formgenauigkeit erreicht, wenn die Einzelteile vor dem Zusammenfügen mechanisch so exakt bearbeitet wurden, dass die erforderlichen engen Passungstoleranzen eingehalten werden.

Das Dichtungselement liegt flächig an Wänden der Absätze der transparenten Elemente an. Um zu verhindern, dass ein Anteil der Probe aufgrund des Kapillareffekts an der Grenzfläche bzw. in möglicherweise vorhandenen kleinen Zwischenräumen zwischen den transparenten Elementen und dem Dichtungselement eindringt, ist vorgesehen, dass das Dichtungselement zwischen den transparenten Elementen in den Probenkanal ragen gelassen wird. Dies wird erzielt, indem ein Dichtungselement aus einem elastischen Material, vorzugsweise mit einer Shore D Härte zwischen 50 und 80, noch bevorzugter mit einer Shore D Härte zwischen 60 und 70, verwendet und dieses Dichtungselement gegen die transparenten Elemente gepresst wird. Aufgrund der Nachgiebigkeit des Materials des Dichtungselements wird dieses in den Spalt zwischen den transparenten Elementen gedrängt und bildet einen Dichtungswulst im Spalt. Die Ausbildung des Dichtungswulstes kann gefördert werden, indem die transparenten Elemente an ihren dem Spalt zugewandten Kanten mit einem Radius oder einer Fase versehen werden. Man vermeidet dadurch so genannte "Probenverschleppung", das ist die Kontaminierung einer Probe durch Reste früherer Proben in der Küvette bzw. das Verfälschen von Referenzmessungen.

Bevorzugt bilden das Dichtungselement und der Abstandshalter ein Kombinationselement, z.B. ein 2-Komponenten-Spritzgussteil oder ein Verbund-Pressteil. Mit einem solchen Kombinationselement wird das Assemblieren der Küvette wesentlich vereinfacht und dennoch hervorragenden Festigkeit und Dichtheit erzielt.

Eine Ausführungsform der Küvette weist transparente Elemente aus Glas, vorzugsweise Pressglas, auf. Diese Ausführungsform zeichnet sich durch ihre gute Herstellbarkeit und hohe Maßhaltigkeit aus.

Alternativ zu Glas kann für die transparenten Elemente Kunststoff mit folgenden Eigenschaften verwendet werden: geringe Spannungsdoppelbrechung, geringes Kriechverhalten, keine/geringe Gasdurchlässigkeit, chemische Resistenz, Wärmeformbeständigkeit, optische Transparenz im sichtbaren (VIS) und nahen Infrarot-(NIR) Wellenlängenbereich. Der sichtbare Bereich (VIS) ist als Wellenlängenbereich zwischen 380 und 780 nm definiert; der nahe Infrarotbereich (NIR) liegt zwischen 780 und 1400 nm. Bevorzugt bestehen die transparenten Elemente aus Kunststoffen aus der Gruppe der thermoplastischen Olefin-Polymere.

Die Auswahl des Materials der transparenten Elemente aus den oben angeführten Materialien lässt auch eine Thermostatisierung der Probe in der Küvette zu. Insbesondere müssen Blutproben während der spektroskopischen Analyse möglichst genau bei 37 °C gehalten werden, da die Spektren temperaturabhängig sind.

Wenn die Küvette in ein Consumable (Verbrauchsgut) des spektroskopischen Analysators, insbesondere in ein Fluidpack, das Funktionsflüssigkeiten (beispielsweise Kalibrationsflüssigkeiten, Referenzflüssigkeiten, Reinigungs- oder Standby-Flüssigkeiten oder auch Reagenzflüssigkeiten) und/oder Abfallbehälter umfasst, integriert ist, wird der Küvettenwechsel auch für den "untrainierten" Benutzer einfach und intuitiv, da er in einem Arbeitsgang mit dem Wechsel des Consumables erfolgt.

Es wurde vom Erfinder überraschend herausgefunden, dass für den erfindungsgemäßen Hämolysator piezoelektrische Multilayer-Aktuatoren hervorragend als mechanische Schwingungserzeugungselemente geeignet sind. Diese Multilayer-Aktuatoren erfüllen die Bedingungen, durch elektrische Wechselsignale in einem breiten Frequenzband durchstimmbar anregbar zu sein. Multilayer-Aktuatoren wurden bereits bei Einspritzsystemen im Automobilbereich verwendet, wo sie aber unter gänzlich anderen Betriebsbedingungen verwendet werden als in den erfindungsgemäßen Hämolysatoren. Die Verwendung von Multilayer-Aktuatoren in medizinischen Geräten zur Hämolyse ist nicht bekannt. Mit piezoelektrischen Multilayer-Aktuatoren können im erfindungsgemäßen Hämolysator große Schwingungsamplituden ohne Anwendung des Resonatorprinzips erreicht werden und die Gesamtbaugröße des Hämolysators kann drastisch reduziert werden.

Die piezolektrischen Multilayer-Aktuatoren sind im erfindungsgemäßen Hämolysator in ein Feder-Masse System integriert, dessen Auslegung an die jeweiligen Bedingungen angepasst werden kann. Die Verwendung von Multilayer-Aktuatoren erfordert spezielle Betriebsbedingungen (Vorspannkräfte, Pulsformen der Ansteuerung). Zur Optimierung ihrer Lebensdauer muss eine "sanfte" und materialschonende Betriebsart und/oder geeignete Materialien gewählt werden. Beispielsweise können hierzu Amplituden, Arbeitsfrequenz, Resonanzfrequenz oder auch verwendete Keramikzusammensetzungen entsprechend ausgewählt werden. Im Gegensatz zum Resonanzprinzip kann auch eine nicht-sinusförmige, optional in der Frequenz durchstimmbare Pulsform zur Hämolysierung des Blutes gewählt werden, wodurch die Hämolyse und die Reinigung des Systems optimiert werden können. Beispiele für anwendbare Signalformen umfassen neben Sinussignalen auch Rechteck-, Dreieck-, Sägezahn- und Impulssignale, es sind aber auch Ansteuersignale mit frei definierbarer Kurvenform zulässig.
Zusammengefasst weist der erfindungsgemäße Hämolysator mit piezoelektrischen Multilayer-Aktuatoren im Vergleich zum bekannten Resonanzschwinger die folgenden Merkmale und Vorteile auf:
1. Durchstimmbares System in Hubamplitude und Hubfrequenz
2. Verbesserung der Hämolysierung und Reinigung mit nicht-sinusförmigen Pulsen und Variationsmöglichkeit der Pulsform, Frequenz und Amplitude
3. Geringere Ansteuerungsspannungen erforderlich
4. Funktionsüberprüfung und Überwachung des Betriebszustandes der Multilayer-Aktuatoren und damit des Hämolysators durch Überwachung der elektrischen Parameter
5. Wesentlich kleinere Bauweise
6. Verwendung der Multilayer-Aktuatoren als Sensor (Kraftmessung, permanente Ermittlung der optimalen Betriebsbedingungen), insbesondere in Kombination mit auswechselbarer Küvette
Ein Problem im Zusammenhang mit Multilayer-Aktuatoren bereitet deren Kontaktierung zur elektrischen Verbindung mit einem elektrischen Signalgenerator. Aufgrund der relativ hohen elektrischen Kapazität der Multilayer-Aktuatoren und deren Ansteuerung mit elektrischen Signalen im kHz-Bereich fließen über die Kontaktierung hohe Ströme (bis 10 A). Dass die Multilayer-Aktuatoren beim Schwingen ihre Dicke verändern, ist eine weitere Schwierigkeit, die bei der Kontaktierung überwunden werden muss. Die vorliegende Erfindung bietet jedoch auch für dieses Problem eine Lösung, die allgemein in Schwingungssystemen mit Multilayer-Aktuatoren, und insbesondere in einem Hämolysator mit Multilayer-Aktuatoren anwendbar ist. Ein beispielhaftes Schwingungssystem mit zumindest einem piezoelektrischen Multilayer-Aktuator zeichnet sich dadurch aus, dass der zumindest eine piezoelektrische Multilayer-Aktuator zwischen einem ersten und einem zweiten Stromleiter eingespannt ist, wobei an den Grenzflächen zwischen dem Multilayer-Aktuator und den Stromleitern elektrisch leitfähige Matten angeordnet sind, die Partikel aus einem elektrisch leitenden Material, insbesondere Kohlenstoff, enthalten.

Die elektrisch leitfähigen Matten mit den Kohlenstoffpartikeln weisen vorzugsweise einen Widerstand mit negativen Temperaturkoeffizienten auf, welche so insbesondere bei steigenden Temperaturen, bedingt durch die Erwärmung des Hämolysators bei dessen Betrieb, eine verbesserte Stromleitung bewirken. Die Kohlenstoffpartikel schmiegen sich eng an die Oberflächen der Stromleiter und der Multilayer-Aktuatoren an, wodurch eine Vergrößerung der für die Stromleitung wirksamen Fläche auf z.B. das Doppelte erzielt wird. Ein weiterer Vorteil ist, dass die elektrisch leitfähigen Matten sehr flexibel sind, so dass - im Gegensatz zu starren Verbindungen, wie Lötverbindungen - auch bei Dickenveränderungen der Multilayer-Aktuatoren eine hervorragende elektrische Leitung aufrecht erhalten wird.
Eine bevorzugte Ausführungsform des erfindungsgemäßen Hämolysators mit einem Schwingungssystem mit piezoelektrischen Multilayer-Aktuatoren ist dadurch gekennzeichnet, dass die piezoelektrischen Multilayer-Aktuatoren zwischen einem ersten und einem zweiten Stromleiter eingespannt sind, wobei an den Grenzflächen zwischen dem zumindest einen Multilayer-Aktuator und den Stromleitern elektrisch leitfähige Matten angeordnet sind, die Partikel aus einem elektrisch leitenden Material, insbesondere Kohlenstoff, enthalten.
Die Erfindung bietet auch ein Verfahren zur Funktionsüberprüfung und Überwachung des Betriebszustandes des mit piezoelektrischen Multilayer-Aktuatoren ausgestatteten Hämolysators durch Überwachung der elektrischen Parameter der Multilayer-Aktuatoren. Eine solche Funktionsüberprüfung und Überwachung ist für den Hämolysator mit austauschbarer Küvette besonders vorteilhaft. Ein ganz wesentlicher Vorteil dieses Verfahrens ist es auch, dass damit in Abhängigkeit von veränderlichen Schwingungseigenschaften (z.B. durch den turnusmäßigen Austausch von Küvetten) ein optimaler Betriebspunkt oder -bereich, beispielsweise ein optimaler Frequenzbereich und/oder eine optimale Hubamplitude, des Hämolysators in Kombination mit einer Küvette gefunden werden kann. Das erfindungsgemäße Verfahren zum Betreiben eines erfindungsgemäßen Hämolysators mit als piezoelektrische Multilayer-Aktuatoren ausgebildeten Schwingungserzeugungselementen basiert darauf, dass die piezoelektrischen Multilayer-Aktuatoren als Sensoren verwendet werden, indem unter Anwendung des Piezoeffekts (d.h. des physikalischen Phänomens, bei dem durch mechanische Druckbelastung auf ein Piezoelement ein elektrisches Signal hervorgerufen wird) physikalische Parameter der Multilayer-Aktuatoren erfasst und ausgewertet werden.

Der erfasste physikalische Parameter ist in einer Ausführungsform des erfindungsgemäßen Verfahrens das Ausschwingen der Sonotrodenplatte nach Abschalten der elektrischen Signalzufuhr, wobei sich das Ausschwingen in der Erzeugung eines elektrischen Spannungssignals durch die Multilayer-Aktuatoren manifestiert. Aus dem von den Multilayer-Aktuatoren erzeugten elektrischen Spannungssignal kann, vorzugsweise mittels Fast Fourier Transformation, eine Mittenfrequenz und optional die Güte des Schwingsystems bestimmt werden.
Alternativ ist der erfasste physikalische Parameter eine auf die Multilayer-Aktuatoren wirkende mechanische Kraft, die durch Messung der von den Multilayer-Aktuatoren generierten elektrischen Spannung ermittelt wird.
Es ist auch vorgesehen, die elektrische Kapazität der Multilayer-Aktuatoren als physikalischen Parameter zu erfassen und auszuwerten.
Wenn sich bei der Auswertung eines erfassten physikalischen Parameter der piezoelektrischen Multilayer-Aktuatoren ergibt, dass er außerhalb eines vorgegebenen Betriebsbereichs liegt, können Fehlerkorrekturmaßnahmen und/oder Alarmierungen eingeleitet werden.
Die Erfindung wird nun anhand von Ausführungsbeispielen unter Bezugnahme auf die Zeichnungen näher erläutert. In den Zeichnungen zeigen:
Fig. 1 ein Prinzipschaubild eines Oxymetermoduls 200 eines spektroskopischen Analysators;
Fig. 2 eine perspektivische Darstellung eines bekannten, auf dem Resonanzschwingerprinzip basierenden Ultraschall-Transducer-Hämolysators;
Fig. 3 einen erfindungsgemäßen Hämolysator als Teil eines spektroskopischen Analysators in der Perspektive;
Fig. 4 eine Draufsicht auf einen erfindungsgemäßen Hämolysator, der mit würfelförmigen piezoelektrischen Multilayer-Aktuatoren ausgebildet ist, die mit leitfähigen Kohlenstoff-Matten kontaktiert sind;
Fig. 5 das dynamische Verhalten eines erfindungsgemäßen Hämolysators in der Zeit- und Frequenzdomäne bei drei verschiedenen Anregungsfrequenzen;
Fig. 6A, Fig. 6B und Fig. 6C eine bevorzugte Ausführungsform einer im erfindungsgemäßen Hämolysator verwendeten Küvette in einer isometrischen Ansicht, in einer Schnittansicht entlang der Linie 2B bzw. in einer Schnittansicht entlang der Linie 2C von Fig. 2A; und
Fig. 7 eine schematische Ansicht eines modular aufgebauten spektroskopischen Analysators, in dem der erfindungsgemäße Hämolysator eingebaut ist.

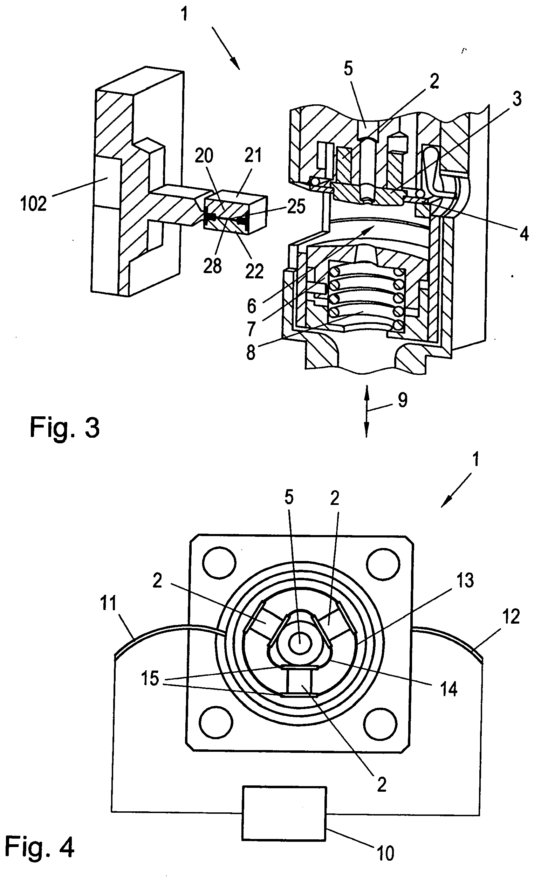

Eine bevorzugte Ausführungsform eines erfindungsgemäßen Hämolysators 1 ist in Fig. 3 und Fig. 4 dargestellt. Der Hämolysator 1 ist in einen spektroskopischen Analysator integriert, der weiter unten näher beschrieben wird. Der Hämolysator 1 umfasst einen Ultraschall-Schwingungsgenerator, der Schwingungserzeugungselemente 2, die von einem nicht dargestellten elektrischen AC-Signalgenerator in einem breiten Frequenzband (z.B. von 20 - 50 kHz) durchstimmbar ansteuerbar sind und eine durch eine Tellerfeder 4 gehaltene Sonotrodenplatte 3 aufweist, auf die die Schwingungserzeugungselemente 2 mechanische Schwingungen in Richtung einer optischen Achse 5 ausüben. Die Sonotrodenplatte 3 überträgt diese mechanischen Schwingungen auf einen Probenraum 6. In den Probenraum 6 ist eine austauschbare Küvette 20 einführbar, die mit einer schwingungsübertragenden Wand 21 (Glaswand, etc.) gegen die Stirnfläche der Sonotrodenplatte 3 anliegt, so dass sie die Schwingungen der Sonotrodenplatte 3 aufnimmt und an eine im Inneren der Küvette vorhandene flüssige Blutprobe 28 weitergibt, wodurch aufgrund von Kavitationseffekten Blasen in der Blutprobe 28 entstehen und zerplatzen, was zur Hämolyse des Blutes führt.

Damit die Schwingungsübertragung auf die Küvette 20 funktioniert, ist auf der der Sonotrodenplatte 3 gegenüberliegenden Seite des Probenraums 6 eine Gegenmasse 7 angeordnet, die nach dem Einführen der Küvette 20 in den Probenraum 6 durch einen nicht näher dargestellten Mechanismus zur Küvette 20 hinbewegt wird und nach dem Durchführen der Messungen wieder zurückbewegt wird (siehe Doppelpfeil 9), um die Küvette freizugeben. Die Gegenmasse 7 presst die Küvette 20 im eingespannten Zustand gegen die Sonotrodenplatte 3. Die Gegenmasse 7 wird von einer Schraubenfeder 8 mechanisch vorgespannt. Die von der Schraubenfeder 8 erzeugte Vorspannkraft soll einerseits gering sein, damit die Küvette 20 nicht zusammengepresst wird, wodurch ihr Probenkanal 25 zusammengequetscht würde, was wiederum die optischen Eigenschaften und damit die Messgenauigkeit verändern würde. Andererseits soll die Vorspannkraft eine Mindestkraft nicht unterschreiten, um zu verhindern, dass die Sonotrodenplatte 3 bei Schwingungen von der Küvette 20 abhebt.

Die Schwingungserzeugungselemente 2 sind als piezoelektrische Multilayer-Aktuatoren (siehe Fig. 4) in Würfelform ausgebildet, die ca. 180 keramische, übereinander gestapelte Schichten aus piezoelektrischem Material umfassen. Jeder Würfel hat eine elektrische Kapazität von ca. 350 - 500 nF. Die Schichten sind mechanisch in Reihe und elektrisch parallel geschaltet, wodurch sie an gegenüberliegenden Rändern seitlich kontaktiert werden und bei Anregung durch ein elektrisches AC-Signal von einem AC-Signalgenerator 10 Längenänderungen in Richtung der optischen Achse 5 erfahren, die sich zu einer Gesamtlängenänderung aufsummieren. Für die Durchführung der Hämolyse ist eine Längenänderung von ca. 1 µm bis ca. 30 µm, vorzugsweise von ca. 5 µm zumeist ausreichend. Die Multilayer-Aktuatoren stützen sich - in Richtung der optischen Achse gesehen - mit einer ersten Stirnfläche gegen einen Trägerkörper des Hämolysators ab und mit der gegenüberliegenden Stirnfläche gegen die Sonotrodenplatte 3. Die Multilayer-Aktuatoren sind vorzugsweise sternförmig um die optische Achse 5 angeordnet. Alternativ wäre das Vorsehen eines koaxial zur optischen Achse angeordneten ringförmigen Multilayer-Aktuators denkbar.

Die Kapazität von ca. 350 - 500 nF pro Multilayer-Aktuator erfordert zu ihrer Ansteuerung Ströme von mehreren Ampere. Um diese Ströme verlässlich in die Multilayer-Aktuatoren einbringen zu können, wurde eine spezifische Kontaktierung entwickelt, die nun anhand der Fig. 4 erläutert wird.

Der Hämolysator 1 weist einen äußeren ringförmigen Stromleiter 13 und einen inneren Dreieck- bzw. Sternförmigen Stromleiter 14 aus Kupfer auf, die mittels Anschlussdrähten 11, 12 an einen elektrischen AC-Signalgenerator 10 angeschlossen sind. Zumindest einer der beiden Stromleiter 13, 14 ist als Klemmfeder ausgebildet. Zwischen den beiden Stromleitern 13, 14 sind die als Multilayer-Aktuatoren ausgebildeten Schwingungserzeugungselemente 2 sternförmig um die optische Achse 5 gruppiert eingespannt, wobei jeweils zwischen einer ersten Seitenwand eines jeden Multilayer-Aktuators und der inneren Mantelfläche des äußeren Stromleiters 13 sowie zwischen einer zweiten Seitenwand eines jeden Multilayer-Aktuators und der äußeren Mantelfläche des inneren Stromleiters 14 eine leitfähige Kontaktierungsmatte 15, insbesondere eine Kohlenstoff/Graphit enthaltende Kontaktierungsmatte 15, angeordnet ist. Diese Kontaktierungsmatten 15 werden aufgrund der mechanischen Klemmkräfte von äußerem und/oder innerem Stromleiter 13, 14 gegen die Seitenwände der Multilayer-Aktuatoren gepresst und dringen dabei in die Rauhigkeiten (Poren) der Wände der Multilayer-Aktuatoren ein, wodurch die Kontaktierungsfläche um ein Vielfaches vergrößert wird. Eine Metallfolie, z.B. Silberfolie, würde diesen Effekt nicht zeigen.

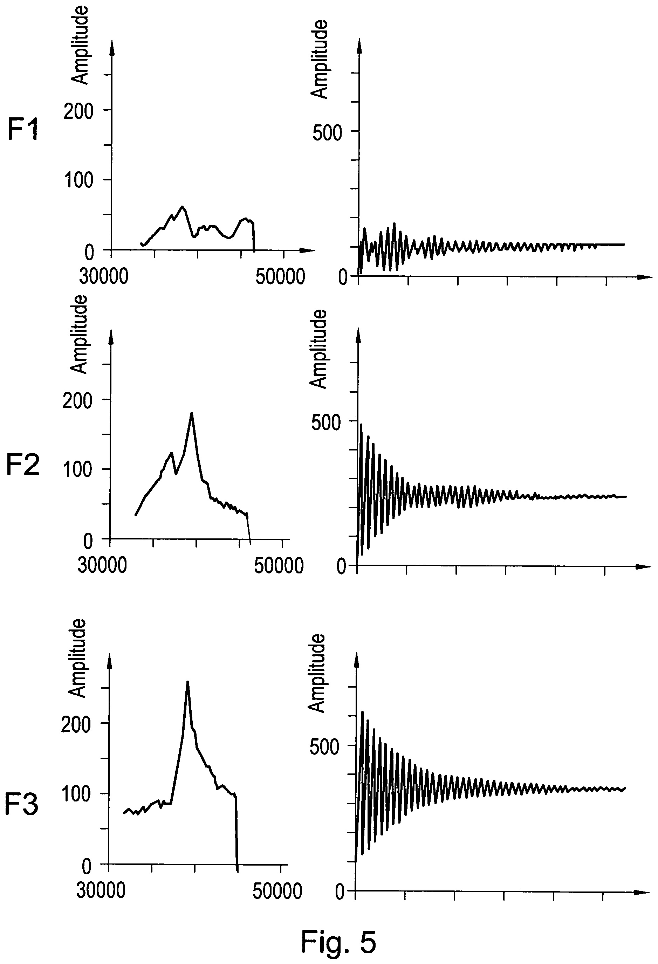

Die Anwendung des in Fig. 4 dargestellten Schwingungssystems mit zumindest einem piezoelektrischen Multilayer-Aktuator als Schwingungserzeugungselement 2, wobei der zumindest eine piezoelektrische Multilayer-Aktuator zwischen einem ersten und einem zweiten Stromleiter 13, 14 eingespannt ist, wobei an den Grenzflächen zwischen dem Multilayer-Aktuator und den Stromleitern 13, 14 elektrisch leitfähige Matten 15 angeordnet sind, die Partikel aus einem elektrisch leitenden Material, insbesondere Kohlenstoff, enthalten, ist nicht auf die Verwendung im Hämolysator eingeschränkt, sondern kann vorteilhaft auch in anderen technischen Gebieten Verwendung finden; dies ist jedoch nicht Teil der vorliegenden Erfindung. Der Hämolysator 1 kann in verschiedenen Betriebsmodi betrieben werden. Die Hauptbetriebsart ist die Hämolyse, wobei am Beginn der Hämolyse der elektrische Signalgenerator 10 bevorzugt eine steile Spannungsrampe an die Schwingungserzeugungselemente 2 (Multilayer-Aktuatoren) anlegt. Es hat sich auch als günstig erwiesen, die Frequenz des elektrischen Signals zu wobbeln, z.B. in einem Bereich von 200 Hz um die Mittenfrequenz, wodurch verbesserte Hämolyseergebnisse erzielt werden können.
Zur Reinigung des Systems können Signale mit stark unterschiedlicher Frequenz an die Schwingungserzeugungselemente 2 angelegt werden, wodurch in der Flüssigkeitsprobe (wobei auch eine spezielle Reinigungsflüssigkeit als Probe vorgesehen werden kann) Blasen mit stark unterschiedlichem Durchmesser und Dichte erzeugt werden, was die Reinigungseffizienz für unterschiedliche Kontaminierungen wesentlich verbessert.
Zur Entfernung von unerwünschten Gasblasen im Probenkanal 25 der Küvette 20 können die Gasblasen in Bewegung versetzt und aus dem Probenkanal 25 transportiert werden, indem ein elektrisches Signal von ca. 2/3 der Mittenfrequenz angelegt wird. Hierzu kann weiterhin das optische Messfenster der Küvette entsprechend geometrisch ausgestaltet sein, daß diese Gasblasen möglichst zuverlässig aus dem optischen Messbereich wegtransportiert werden.
Weiters können zur Durchführungen von Überwachungen und Qualitätskontrollen die in Form von piezoelektrischen Multilayer-Aktuatoren ausgebildeten Schwingungserzeugungselemente 2 als Sensoren verwendet werden, indem unter Anwendung des piezoelektrischen Effekts physikalische Parameter der Multilayer-Aktuatoren erfasst und ausgewertet werden. Damit ist auch das dynamische Verhalten des Hämolysators 1 bzw. des Gesamt-Schwingungssystems im Hämolysator erfassbar. Dazu wird beispielsweise der Ausschwingvorgang der Sonotrodenplatte 2 nach Abschalten der elektrischen Signalzufuhr zu den Multilayer-Aktuatoren erfasst, wobei sich das Ausschwingen in der Erzeugung eines elektrischen Spannungssignals durch die Multilayer-Aktuatoren manifestiert. Aus den von den Multilayer-Aktuatoren erzeugten elektrischen Spannungssignalen werden mittels Fast Fourier Transformation die Mittenfrequenz und optional die Güte des Schwingsystems bestimmt. Fig. 5 zeigt das dynamische Verhalten des Hämolysators 1 in der Zeit (rechts, Einheit der x-Achse in rel. Zeiteinheiten, Einheit der y-Achse in mV)- und Frequenzdomäne (links, Einheit der x-Achse in Hz, Einheit der y-Achse in rel. Einheiten) bei drei verschiedenen Anregungsfrequenzen F1, F2, F3. Der obere Kurvenverlauf (Anregungsfrequenz F1) zeigt eine Fehlanpassung der Frequenz des elektrischen Signalgenerators 10, der mittlere Kurvenverlauf (Anregungsfrequenz F2) zeigt eine fast korrekte Einstellung der Frequenz des elektrischen Signalgenerators 10, und der untere Kurvenverlauf (Anregungsfrequenz F3) zeigt eine optimale Einstellung der Frequenz des elektrischen Signalgenerators 10.

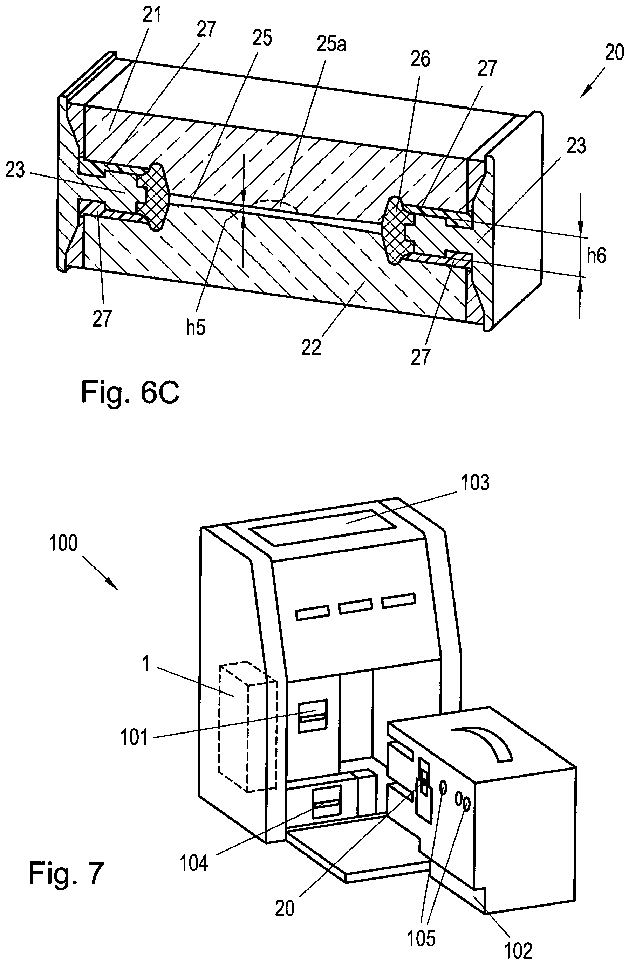

Fig. 7 zeigt schematisch ein modulares Konzept eines spektroskopischen Analysators 100, in dem der oben beschriebene Hämolysator 1 in Kombination mit der Küvette 20 verwendet wird. Der Analysator 100 ist als "beinahe wartungsfrei" ausgelegt, so dass alle zum laufenden Betrieb benötigten Verbrauchsmaterialien in Form von Kassetten und/oder Modulen (so genannten "Consumables") vorliegen und daher auch von (technisch) ungeschultem Personal getauscht werden können. Die verwendeten Verbrauchsmaterialien sind in diesem Ausführungsbeispiel in folgenden Consumables zusammengefasst:
- Eine Sensorkassette 101, welche zumindest einen Teil, vorzugsweise alle der für die Analytbestimmung benötigten Sensoren enthält
- Ein Fluidpack 102, das Flüssigkeitsbehälter, Reagenzienpacks und Abfallbehälter enthält, welche die zum Betrieb des Analysators 100 benötigten Funktionsfluide (z.B. Kalibrationslösungen, Waschlösungen, Referenzflüssigkeiten, bestimmte zum Betrieb benötigte Reagenzienlösungen...) enthält. Optional können im Fluidpack 102 auch weitere Elemente oder Funktionalitäten wie das gesamte Fluidiksystem oder Teile davon, die Probeneingabevorrichtung oder auch weitere sensorische Komponenten enthalten sein. In das Fluidpack 102 ist eine Küvette 20 inkl. der zugehörigen flüssigkeitszu- und -abführenden Fluidikwege integriert, wie weiter unten näher ausgeführt wird. Das heißt, dass die Küvette 20 turnusmäßig mit jedem Austausch des Fluidpacks 102 getauscht wird (z.B. in Abständen von mehreren Wochen)
- Eine Druckerpapierkassette 103 für einen internen Drucker
- Optional eine Qualitätskontrollkassette 104 mit Referenzlösungen in Ampullenform zur Durchführungen einer automatisierten Qualitätskontrolle, welche das Personal durch einfache intuitive Handgriffe selbst tauschen kann.

Die hier geschilderte Untergliederung der Consumables ist nur exemplarisch. Es ist auch denkbar, dass (Teil-)Funktionalitäten oder (Teil-)Elemente mehrerer Consumables zusammengefasst werden, so dass beispielsweise weniger oder sogar nur ein Consumable benötigt wird. Andererseits ist es auch denkbar, dass (Teil-)Funktionalitäten oder (Teil-)Elemente einzelner Consumables auf mehrere verteilt werden (z.B. auf mehrere Sensorkassetten oder -module). Wesentlich ist aber der Grundgedanke, in eines der verwendeten Consumables die Küvette einzubauen, so dass sie zusammen mit diesem Consumable tauschbar ist.

Die Consumables werden untereinander bzw. mit dem Analysator durch aufeinander abgestimmte Schnittstellen, z.B. in Form fluidischer Andocknippel 105 gekoppelt. Das mechanische Verbinden der Consumables mit den respektiven Gegenparts kann durch einen einfachen manuellen Bewegungsablauf direkt durch den Benutzer erfolgen, oder durch im Gerät befindliche Antriebe, die die Ankopplung automatisch durchführen, nachdem der Benutzer die Kassette nur in "Position" gebracht hat.

Der Blutgas-Analysator 100 enthält ein Oxymetermodul, in dem durch ein spektrophotometrisches Messverfahren die Konzentrationen der Hämoglobinderivate O₂Hb, HHb, COHb, MetHb, sowie der Blutparameter tHb (total hemoglobin), SO₂ (oxygen saturation) und Bilirubin von der in der Küvette 20 befindlichen bestimmt werden. Dabei werden charakteristische Absorptionseigenschaften dieser Substanzen genutzt und die Messwerte über einen mathematischen Algorithmus ausgewertet. Um die geforderte Messgenauigkeit erreichen zu können, ist zumeist eine Hämolyse des Vollblutes vor der optischen Messung notwendig. Zur Durchführung der Hämolyse ist der schematisch angedeutete Hämolysator 1 in das Oxymetermodul integriert. Das Oxymetermodul beinhaltet weiters - ähnlich wie in Fig. 1 dargestellt - eine Lampeneinheit mit Lichtquelle(n), fluidische Zufuhr- und Abfuhrleitungen, einen Lichtleiter, der das in der Lampeneinheit erzeugte Licht zur Küvette 20 zuführt und einen Lichtleiter, der das durch die Probe in der Küvette 20 hindurchgegangene Licht sammelt und an einen Polychromator weiterleitet, der eine spektrale Auftrennung des empfangenen Lichts vornimmt, sowie einen Detektor zur Auswertung der spektralen Bereiche des empfangenen Lichts. Der Hämolysator 1 ist so ausgestaltet, dass die Küvette 20 als Teil des Consumables 102 beim Einführen des Consumables 102 in den Analysator 100 in den Hämolysator 1 eingesetzt wird und beim Herausziehen des Consumables 102 aus dem Analysator 100 aus dem Hämolysator 1 entfernt wird. Durch diese Konstruktion vermeidet man die Verstopfungsprobleme an Oxymetermodulen bekannter Analysatoren, bei denen eine optische Messkammer (Küvette) als integriertes Bestandteil des Analysators ausgeführt ist, welches permanent im Gerät verbleibt.

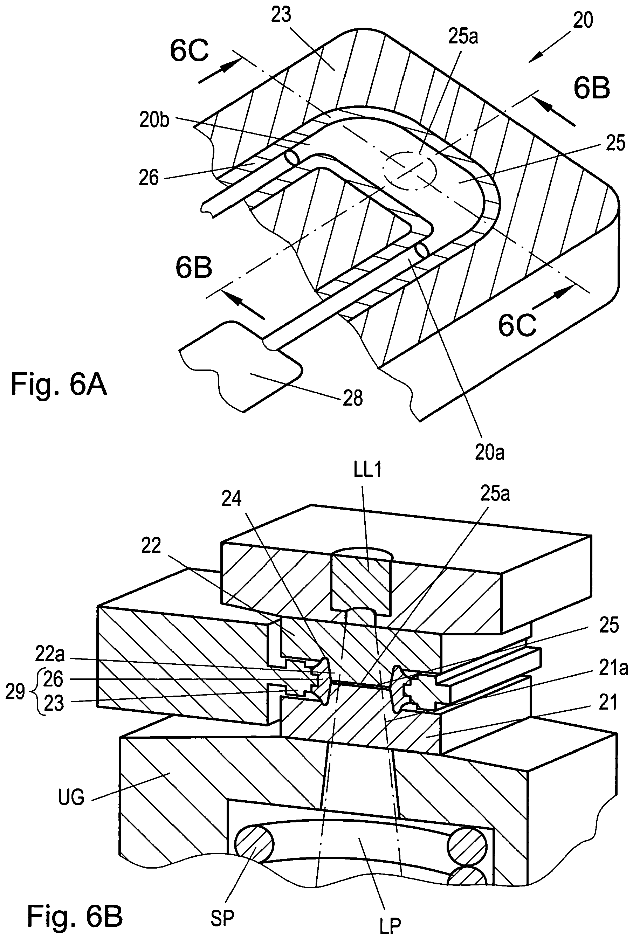

Anhand von Fig. 6A, Fig. 6B und Fig. 6C wird nun die Ausführung der Küvette 20 erklärt. Die Küvette 20 weist einen Abstandshalter 23, ein erstes und ein zweites transparentes Element 21, 22 und ein Dichtungselement 26 auf. Die beiden transparenten Elemente 21, 22 weisen jeweils einen Absatz 21a, 22a auf und sind einander gegenüberliegend so an dem Abstandshalter 23 angeordnet, dass die beiden Absätze 21a, 22a einander zugewandt sind und sich in eine kanalförmige Aussparung 24 erstrecken. Dabei weisen die Stirnflächen der Absätze 21a, 22a einen solchen Abstand voneinander auf, der einer definierten Höhe eines zwischen den Stirnflächen der Absätze 21a, 22a gebildeten Probenkanals 25 entspricht. Der Probenkanal 25 wird um seinen Umfang herum von einem Dichtungselement 26 abgedichtet, wobei das Dichtungselement 26 an den Mantelflächen der Absätze 21a, 22a anliegt und in den Spalt dazwischen drängt. Das Dichtungselement 26 mit dem Abstandshalter 23 ist als ein Kombinationselement 29, z.B. ein 2-Komponenten-Spritzgussteil oder ein Verbund-Pressteil, gebildet. Dies bringt wesentliche Vorteile beim Assemblieren der Küvette 20. Damit die Küvette 20 für Ultraschall-Applikationen geeignete Festigkeit aufweist, ist der Abstandshalter 23 aus einem spritzgussfähigen Kunststoff mit hohen E-Modulwerten von vorzugsweise mehr als 2500 MPa, noch bevorzugter mehr als 5000 MPa, gefertigt.

Die transparenten Elemente 21, 22 sind aus Glas, vorzugsweise Pressglas, hergestellt, das sich gut bearbeiten lässt. Alternativ sind sie aus einem Kunststoff gefertigt, der folgende Eigenschaften aufweist: geringe Spannungsdoppelbrechung, geringes Kriechverhalten, keine/geringe Gasdurchlässigkeit, chemische Resistenz, Wärmeformbeständigkeit, optische Transparenz im sichtbaren (VIS) und nahen Infrarot- (NIR) Wellenlängenbereich. Der sichtbare Bereich (VIS) ist als Wellenlängenbereich zwischen 380 und 780 nm definiert; der nahe Infrarotbereich (NIR) liegt zwischen 780 und 1400 nm. Bevorzugt bestehen die transparenten Elemente aus Kunststoffen aus der Gruppe der thermoplastischen Olefin-Polymere.

Das Dichtungselement 26 besteht aus einem Elastomer, vorzugsweise mit einer Shore D Härte zwischen 50 und 80, noch bevorzugter mit einer Shore D Härte zwischen 60 und 70.

Der Probenkanal 25 weist einen optischen Messbereich 25a auf, in dem die transparenten Elemente 21, 22 planparallel angeordnet sind. Zur Vermeidung von Randeffekten ist der optische Messbereich 25a vom Rand des Probenkanals 25 beabstandet. Nahe dem Messbereich 25a mündet ein Lichtleiter LL1, der Licht durch den Messbereich 25a entlang des Lichtpfads LP ausstrahlt, das nach seinem Durchgang durch den Messbereich 25 spektroskopisch analysiert wird. Alternativ dazu sind auch reflektive optische Messsysteme vorgesehen.
Der Probenkanal 25 ist bogenförmig ausgebildet, so dass die Einlassöffnung 20a für die Probe 28 und die Auslassöffnung 20b auf derselben Seite liegen, was den Vorteil eines verringerten Bauvolumens mit sich bringt. Zusätzlich kann so die austauschbare Einsetzbarkeit der Küvette erleichtert werden, da alle fluidischen Anschlüsse auf einer Seite liegen. Weiters ist zu beachten, dass sich von einem zentralen Bereich, der den Messbereich 25a umfasst, zur Einlassöffnung 20a und zur Auslassöffnung 20b hin die Breite des Probenkanals 25 verjüngt, dabei aber die Probenkanalhöhe zunimmt, so dass die Querschnittsfläche des Probenkanals 25 über seine Länge im Wesentlichen konstant bleibt. Man unterbindet damit Wirbelbildung der Probe 28 im Probenkanal 25. Für denselben Zweck weist der Probenkanal 25 nur stetige Kanalveränderungen auf.
Beim Assemblieren der Küvette 20 können die beiden transparenten Elemente 21, 22 mit dem Abstandshalter 23 verklebt werden. Als Alternative bietet sich bei dieser Ausführungsform auch Verpressen oder Stecken an.
In Fig. 6C, die einen Längsschnitt durch den Probenkanal 25 zeigt, ist am besten zu sehen, dass der Probenkanal 25 sich zum Messbereich 25a keilförmig verjüngende Einlaufschrägen aufweist und im Messbereich 25 einen planparallelen Verlauf der den Probenkanal definierenden Flächen der transparenten Elemente 21, 22 aufweist. Weiters sind in Fig. 6C auch die Kleberschichten 27 zu sehen, mit denen der Abstandshalter 23 mit den beiden transparenten Elementen 21, 22 verklebt ist. Der Kleber ist ein nach dem Härten formstabiler Kleber, so dass die Kleberschichten 27 eine definierte Dicke aufweisen.

## Patentansprüche

1. Verfahren zum Betreiben eines Hämolysators (1), wobei der Hämolysator (1) eine Sonotrode (3) und darauf wirkende, als piezoelektrische Multilayer-Aktuatoren ausgebildete Schwingungserzeugungselemente (2) aufweist, die von einem elektrischen AC-Signalgenerator (10) in einem breiten Frequenzband von 20 - 50 kHz durchstimmbar und optional in ihrer Hubamplitude verstellbar zu mechanischen Schwingungen anregbar sind, wobei der Hämolysator (1) einen Probenraum (6) aufweist, auf den die Sonotrode (3) mechanische Schwingungen überträgt, wobei die piezoelektrischen Multilayer-Aktuatoren als Sensoren verwendet werden, indem unter Anwendung des Piezoeffekts physikalische Parameter der Multilayer-Aktuatoren erfasst und ausgewertet werden, wobei in den Probenraum (6) eine austauschbare Küvette (20) eingesetzt wird, die von einer schwingungsübertragenden Wand begrenzt wird, die im eingesetzten Zustand der Küvette (20) gegen die Sonotrode anliegt, **dadurch gekennzeichnet, dass** die Sonotrode als Sonotrodenplatte (3) ausgebildet ist, dass die austauschbare Küvette (20) einen Probenkanal (25) zur Aufnahme einer Blutprobe (28) aufweist, der durch zumindest eine schwingungsübertragende Wand begrenzt wird, die im eingesetzten Zustand der Küvette (20) gegen die Sonotrodenplatte (3) anliegt, und dass der Hämolysator (1) mit der Küvette (20) kombiniert wird, wobei die Küvette (20) mit zumindest einem Dichtungselement (26) und zwei transparenten Elementen (21, 22) versehen ist, wobei die beiden transparenten Elemente (21, 22) im Abstand voneinander angeordnet sind und zwei einander gegenüberliegende Begrenzungsflächen eines Probenkanals (25) definieren und das zumindest eine Dichtungselement (26) Seitenwände des Probenkanals (25) definiert, wodurch der Probenkanal (25) als ein in Längsrichtung geschlossener Kanal mit einer Einlassöffnung (20a) und einer Auslassöffnung (20b) ausgebildet ist, wobei zumindest ein Abstandshalter (23) vorgesehen ist, der die transparenten Elemente (21, 22) im Abstand voneinander hält, und zumindest eines der beiden transparenten Elemente (21, 22) einen Absatz (21a, 22a) aufweist, der sich in Richtung zum anderen transparenten Element erstreckt und eine Begrenzungsfläche des Probenkanals (25) bildet, so dass die Höhe (h5) des Probenkanals geringer ist als die Höhe (h6) des zumindest einen Abstandshalters (23).

2. Verfahren nach Anspruch 1, **dadurch gekennzeichnet, dass** der erfasste physikalische Parameter das Ausschwingen der Sonotrodenplatte (3) nach Abschalten der elektrischen Signalzufuhr ist, wobei sich das Ausschwingen in der Erzeugung eines elektrischen Spannungssignals durch die Multilayer-Aktuatoren manifestiert.

3. Verfahren nach Anspruch 2, **dadurch gekennzeichnet, dass** aus dem von den Multilayer-Aktuatoren erzeugten elektrischen Spannungssignal, vorzugsweise mittels Fast Fourier Transformation, eine Mittenfrequenz und optional die Güte des Schwingsystems bestimmt wird.

4. Verfahren nach Anspruch 1, **dadurch gekennzeichnet, dass** der erfasste physikalische Parameter eine auf die Multilayer-Aktuatoren wirkende mechanische Kraft ist, die durch Messung der von den Multilayer-Aktuatoren generierten elektrischen Spannung ermittelt wird.

5. Verfahren nach Anspruch 1, **dadurch gekennzeichnet, dass** der erfasste physikalische Parameter die elektrische Kapazität der Multilayer-Aktuatoren ist.

6. Verfahren nach Anspruch 1, **dadurch gekennzeichnet, dass** wenn ein erfasster physikalischer Parameter der piezoelektrischen Multilayer-Aktuatoren außerhalb eines vorgegebenen Betriebsbereichs liegt, Fehlerkorrekturmaßnahmen und/oder Alarmierungen eingeleitet werden.

7. Hämolysator (1), mit einer Sonotrode (3) und darauf wirkenden, als piezoelektrische Multilayer-Aktuatoren ausgebildeten Schwingungserzeugungselementen (2), wobei die Schwingungserzeugungselemente (2) von einem elektrischen AC-Signalgenerator (10) in mechanische Schwingungen versetzbar sind, und mit einem Probenraum (6), auf den die Sonotrode (3) mechanische Schwingungen überträgt, wobei die Schwingungserzeugungselemente (2) in einem breiten Frequenzband von 20 - 50 kHz durchstimmbar zu mechanischen Schwingungen anregbar sind, wobei der Hämolysator dazu ausgebildet ist, die piezoelektrischen Multilayer-Aktuatoren als Sensoren zu verwenden, indem er unter Anwendung des Piezoeffekts physikalische Parameter der Multilayer-Aktuatoren erfasst und auswertet, und wobei in dem Probenraum (6) eine austauschbare Küvette (20) vorgesehen ist, die von einer schwingungsübertragenden Wand begrenzt wird, die im eingesetzten Zustand der Küvette (20) gegen die Sonotrode anliegt, **dadurch gekennzeichnet, dass** die Sonotrode als Sonotrodenplatte (3) ausgebildet ist, dass die austauschbare Küvette (20) einen Probenkanal (25) zur Aufnahme einer Blutprobe (28) aufweist, der durch zumindest eine schwingungsübertragende Wand begrenzt wird, die im eingesetzten Zustand der Küvette (20) gegen die Sonotrodenplatte (3) anliegt, und dass der Hämolysator (1) mit der Küvette (20) kombiniert ist, wobei die Küvette (20) mit zumindest einem Dichtungselement (26) und zwei transparenten Elementen (21, 22) versehen ist, wobei die beiden transparenten Elementen (21, 22) im Abstand voneinander angeordnet sind und zwei einander gegenüberliegende Begrenzungsflächen eines Probenkanals (25) definieren und das zumindest eine Dichtungselement (26) Seitenwände des Probenkanals (25) definiert, wodurch der Probenkanal (25) als ein in Längsrichtung geschlossener Kanal mit einer Einlassöffnung (20a) und einer Auslassöffnung (20b) ausgebildet ist, wobei zumindest ein Abstandshalter (23) vorgesehen ist, der die transparenten Elemente (21, 22) im Abstand voneinander hält, und zumindest eines der beiden transparenten Elemente (21, 22) einen Absatz (21a, 22a) aufweist, der sich in Richtung zum anderen transparenten Element erstreckt und eine Begrenzungsfläche des Probenkanals (25) bildet, so dass die Höhe (h5) des Probenkanals geringer ist als die Höhe (h6) des zumindest einen Abstandshalters (23).

8. Hämolysator mit austauschbarer Küvette nach Anspruch 7, **dadurch gekennzeichnet, dass** die Schwingungserzeugungselemente (2) in ihrer Hubamplitude verstellbar zu mechanischen Schwingungen anregbar sind.

9. Hämolysator mit austauschbarer Küvette nach Anspruch 7, **dadurch gekennzeichnet, dass** die Küvette (20) in ein Consumable (102) eines spektroskopischen Analysators (100), insbesondere in ein Fluidpack, das Funktionsflüssigkeiten und/oder Abfallbehälter umfasst, integriert ist.

10. Hämolysator mit austauschbarer Küvette nach Anspruch 7 in Kombination mit einem elektrischen AC-Signalgenerator (10) zur Anregung der Schwingungserzeugungselemente (2), **dadurch gekennzeichnet, dass** der elektrische AC-Signalgenerator (10) nicht-sinusförmige Signale, z.B. Signale mit freidefinierbaren Kurvenformen und/oder in der Frequenz durchstimmbare Signale erzeugt.

11. Hämolysator mit austauschbarer Küvette nach Anspruch 7 oder 10, **dadurch gekennzeichnet, dass** die Schwingungserzeugungselemente (2) zwischen einem ersten und einem zweiten Stromleiter (13, 14) eingespannt sind, wobei an den Grenzflächen zwischen dem zumindest einen Schwingungserzeugungselement (2) und den Stromleitern elektrisch leitfähige Matten (15) angeordnet sind, die Partikel aus einem elektrisch leitenden Material, insbesondere Kohlenstoff, enthalten.

12. Hämolysator mit austauschbarer Küvette nach Anspruch 7, **dadurch gekennzeichnet, dass** die als Sensoren verwendeten piezoelektrischen Multilayer-Aktuatoren so ausgebildet sind, dass mit ihren Signalen ein optimaler Frequenzbereich und/oder eine optimale Hubamplitude für die Schwingungserzeugungselemente des Hämolysators in Kombination mit einer Küvette festlegbar sind.

13. Spektroskopischer Analysator (100) zur spektroskopischen Analyse einer in einer zumindest teilweise transparenten Küvette (20) befindlichen Probe (28), wie z.B. einer Vollblutbrobe, durch Bestrahlen der Probe mit einem Lichtstrahl und Erfassen des Spektrums des Lichtstrahls nach seinem Hindurchgang durch die Probe, wobei der Analysator dazu ausgebildet ist, vor der Bestrahlung der Probe mit einem Lichtstrahl eine Hämolyse der Probe durchzuführen, **dadurch gekennzeichnet, dass** der Analysator zur Durchführung der Hämolyse einen Hämolysator (1) mit austauschbarer Küvette nach einem der vorhergehenden Ansprüchen 7 bis 12 aufweist.

## Claims

1. A method for operating a hemolyser (1), wherein the hemolyser (1) has a sonotrode (3) and vibration generating elements (2) configured as piezo-electric multi-layered actuators acting thereon, which may be tuned by an electric AC signal generator (10) in a wide frequency band of 20 - 50 kHz and optionally excited in the stroke amplitude thereof adjustably to mechanical vibrations, wherein the hemolyser (1) has a sample room (6), onto which the sonotrode (3) transfers mechanical vibrations, wherein the piezo-electric multi-layered actuators are used as sensors, by physical parameters of the multi-layered actuators being recorded and evaluated using the piezoelectric effect, wherein into the sample room (6) there is inserted a replacable cuvette (20), which is limited by the vibration transferring wall, which in the inserted condition of the cuvette (20) rests against the sonotrode, **characterized in that** the sonotrode is configured as a sonotrode plate (3), that the replacable cuvette (20) has a sample channel (25) for receiving a blood sample (28), which is limited by at least one vibration transferring wall, which in the inserted condition of the cuvette (20) rests against the sonotrode plate (3), and that the hemolyser (1) is combined with the cuvette (20), wherein the cuvette (20) is provided with at least one sealing element (26) and two transparent elements (21, 22), wherein the two transparent elements (21, 22) are arranged spaced apart from each other and define two opposite bounding surfaces of a sample channel (25) and the at least one sealing element (26) defines side walls of the sample channel (25), whereby the sample channel (25) is configured as a channel closed in the longitudinal direction having an inlet opening (20a) and an outlet opening (20b), wherein there is provided at least one spacer (23), which spaces apart the transparent elements (21, 22) in a distance to each other, and wherein at least one of the two transparent elements (21, 22) has a platform (21a, 22a) extending in the direction to the other transparent element and forming a boundary surface of the sample channel (25) such that the height (h5) of the sample channel is smaller than the height (h6) of the at least one spacer (23).

2. A method according to claim 1, **characterized in that** the recorded physical parameter is the settling of the sonotrode plate (3) upon switching off the electric signal supply, wherein the settling is manifested in the generation of an electric voltage signal by the multi-layered actuators.

3. A method according to claim 2, **characterized in that** on the basis of the electric voltage signal generated by the multi-layered actuators there is determined, preferably by means of Fast Fourier Transformation, a mid-band frequency and optionally the performance of the swinging system.

4. A method according to claim 1, **characterized in that** the recorded physical parameter is a mechanical force acting on the multi-layered actuators, which is determined by measurement of the electric voltage generated by the multi-layered actuators.

5. A method according to claim 1, **characterized in that** the recorded physical parameter is the electric capacity of the multi-layered actuators.

6. A method according to claim 1, **characterized in that**, if a recorded physical parameter of the piezo-electric multi-layered actuators lies without a predetermined operational range, then there will be introduced error correction measures and/or alerting signals.

7. A hemolyser (1) having a sonotrode (3) and vibration generating elements (2) configured as piezo-electric multi-layered actuators acting thereon, wherein the vibration generating elements (2) may be set by an electric AC signal generator (10) into mechanical vibrations, and having a sample room (6), onto which the sonotrode (3) transfers mechanical vibrations, wherein the vibration generating elements (2) may be tuneably excited in a wide frequency band of 20 - 50 kHz to mechanical vibrations, wherein the hemolyser is configured to use the piezo-electric multi-layered actuators as sensors, by recording and evaluating physical parameters of the multi-layered actuators using the piezo-electric effect, and wherein in the sample room (6) there is provided a replaceable cuvette (20), which is limited by the vibration transferring wall, which in the inserted condition of the cuvette (20) rests against the sonotrode, **characterized in that** the sonotrode is configured as a sonotrode plate (3), that the replaceable cuvette (20) has a sample channel (25) for receiving a blood sample (28), which is limited by a vibration transferring wall, which in the inserted condition of the cuvette (20) rests against the sonotrode (3), and that the hemolyser (1) is combined with the cuvette (20), wherein the cuvette (20) is provided with at least one sealing element (26) and two transparent elements (21, 22), wherein the two transparent elements (21, 22) are arranged spaced apart from each other and define two opposite bounding surfaces of a sample channel (25) and the at least one sealing element (26) defines side walls of the sample channel (25), whereby the sample channel (25) is configured as a channel closed in the longitudinal direction having an inlet opening (20a) and an outlet opening (20b), wherein there is provided at least one spacer (23), which spaces apart the transparent elements (21, 22) in a distance to each other, and wherein at least one of the two transparent elements (21, 22) has a platform (21a, 22a) extending in the direction to the other transparent element and forming a boundary surface of the sample channel (25) such that the height (h5) of the sample channel is smaller than the height (h6) of the at least one spacer (23).

8. A hemolyser having a replaceable cuvette according to claim 7, **characterized in that** the vibration generating elements (2) may be excited in the stroke amplitude thereof adjustable to mechanical vibrations.

9. A hemolyser having a replaceable cuvette (20) according to claim 7, **characterized in that** the cuvette (20) is integrated in a consumable (102) of a spectroscopic analyser (100), in particular in a fluid pack comprising functional liquids and/or waste containers.

10. A hemolyser having a replacable cuvette according to claim 7 in combination with an electric AC signal generator (10) for exciting the vibration generating elements (2), **characterized in that** the electric AC signal generator (10) generates non-sinus-like signals, e.g., signals having freely definable curve shapes and/or signals tuneable in frequency.

11. A hemolyser having a replacable cuvette according to claim 7 or 10, **characterized in that** the vibration generating elements (2) are clamped between a first and second conductor (13, 14), wherein at the boundary surfaces between the at least one vibration generating element (2) and the conductors there are arranged electrically conductive pads (15) containing particles made from an electrically conductive material, in particular carbon.

12. A hemolyser having a replacable cuvette according to claim 7, **characterized in that** the piezo-electric multi-layered actuators used as sensors are configured such that by means of the signals thereof there may be determined an optimal frequency range and/or an optimal stroke amplitude for the vibration generating elements of the hemolyser in combination with a cuvette.

13. A spectroscopic analyser (100) for the spectroscopic analysis of a sample (28) disposed in an at least in part transparent cuvette (20), such as, e.g., a whole-blood sample, by irradiating the sample with a ray of light and recording the spectrum of the ray of light after having passed the sample, wherein the analyser is configured to carry out a hemolysis of the sample before the irradiation of the sample with a ray of light, **characterized in that** the analyser for carrying out the hemolysis has a hemolyser (1) having a replaceable cuvette according to any of the preceding claims 7 to 12.

## Revendications

1. Procédé pour le fonctionnement d'un hémolysateur (1), le hémolysateur (1) comprenant une sonotrode (3) sur laquelle agissent des éléments de production de vibrations (2), qui sont réalisés sous la forme d'actionneurs multicouches piézoélectriques qui peuvent être excités par un générateur électrique de signaux en courant alternatif (10) de manière à obtenir des vibrations mécaniques avec possibilité d'accordage dans une large bande de fréquences de 20 à 50 kHz et éventuellement ayant leur amplitude de course ajustable, l'hémolysateur(1) comprenant une chambre à échantillons (6) à laquelle la sonotrode (3) transmet des vibrations mécaniques, les actionneurs multicouches piézoélectriques étant utilisés comme capteurs, par le fait que les paramètres physiques des actionneurs multicouches sont détectés et évalués au moyen de l'effet piézoélectrique, une cuvette (20) interchangeable étant insérée dans la chambre à échantillons (6) et étant délimitée par une paroi transmettant les vibrations, qui s'applique contre la sonotrode une fois la cuvette (20) insérée, **caractérisé en ce que** la sonotrode est réalisée sous la forme d'une plaque de sonotrode (3), **en ce que** la cuvette (20) interchangeable comprend un canal d'échantillonnage (25) pour la réception d'un échantillon de sang (28), lequel canal est délimité par au moins une paroi transmettant les vibrations qui s'applique contre la plaque de sonotrode (3) une fois la cuvette (20) insérée, et **en ce que** l'hémolysateur (1) est combiné à la cuvette (20), la cuvette (20) étant pourvue d'au moins un élément d'étanchéité (26) et de deux éléments transparents (21, 22), les deux éléments transparents (21, 22) étant agencés à distance l'un de l'autre et définissant deux surfaces de délimitation opposées l'une à l'autre d'un canal d'échantillonnage (25) et l'au moins un élément d'étanchéité (26) définissant les parois latérales du canal d'échantillonnage (25), ce qui a pour effet que le canal d'échantillonnage (25) est réalisé sous la forme d'un canal fermé dans la direction longitudinale pourvu d'un orifice d'entrée (20a) et d'un orifice de sortie (20b), au moins un espaceur (23) étant prévu, qui maintient les éléments transparents (21, 22) à distance l'un de l'autre, et au moins l'un des deux éléments transparents (21, 22) présentant une partie en retrait (21a, 22a) qui s'étend en direction de l'autre élément transparent et qui forme une surface de délimitation du canal d'échantillonnage (25), de sorte que la hauteur (h5) du canal d'échantillonnage est inférieure à la hauteur (h6) de l'espaceur ou des espaceurs (23).

2. Procédé selon la revendication 1, **caractérisé en ce que** le paramètre physique détecté est l'amortissement des vibrations de la plaque de sonotrode (3) une fois l'amenée des signaux électriques interrompue, l'amortissement des vibrations se manifestant par la génération d'un signal de tension électrique par les actionneurs multicouches.

3. Procédé selon la revendication 2, **caractérisé en ce qu'**une fréquence moyenne et éventuellement la qualité du système de vibrations sont déterminées à partir du signal de tension électrique produit par les actionneurs multicouches, de préférence au moyen d'une transformée de Fourier.

4. Procédé selon la revendication 1, **caractérisé en ce que** le paramètre physique détecté est une force mécanique agissant sur les actionneurs multicouches, qui est déterminée par mesure de la tension électrique générée par les actionneurs multicouches.

5. Procédé selon la revendication 1, **caractérisé en ce que** le paramètre physique détecté est la capacité électrique des actionneurs multicouches.

6. Procédé selon la revendication 1, **caractérisé en ce que** lorsqu'un paramètre physique détecté des actionneurs multicouches piézoélectriques se situe à l'extérieur d'une plage de fonctionnement prédéfinie, des mesures de correction d'erreur et/ou des alarmes sont déclenchées.

7. Hémolysateur (1), comprenant une sonotrode (3) sur laquelle agissent des éléments de production de vibrations (2), qui sont réalisés sous la forme d'actionneurs multicouches piézoélectriques, les éléments de production de vibrations (2) pouvant être mis en vibration mécanique par un générateur de signaux électriques de courant alternatif (10), et comprenant une chambre à échantillons (6) à laquelle la sonotrode (3) transmet des vibrations mécaniques, les éléments de production de vibrations (2) pouvant être excités de manière à obtenir des vibrations mécaniques avec possibilité d'accordage dans une large bande de fréquences de 20 à 50 kHz, l'hémolysateur étant conçu pour utiliser les actionneurs multicouches piézoélectriques comme capteurs en détectant et en évaluant les paramètres physiques des actionneurs multicouches au moyen de l'effet piézoélectrique, une cuvette (20) interchangeable étant prévue dans la chambre à échantillons (6) et étant délimitée par une paroi transmettant les vibrations, qui s'applique contre la sonotrode une fois la cuvette (20) insérée, **caractérisé en ce que** la sonotrode est réalisée sous la forme d'une plaque de sonotrode (3), **en ce que** la cuvette (20) interchangeable comprend un canal d'échantillonnage (25) pour la réception d'un échantillon de sang (28), lequel canal est délimité par au moins une paroi transmettant les vibrations qui s'applique contre la plaque de sonotrode (3) une fois la cuvette (20) insérée, et **en ce que** l'hémolysateur (1) est combiné à la cuvette (20), la cuvette (20) étant pourvue d'au moins un élément d'étanchéité (26) et de deux éléments transparents (21, 22), les deux éléments transparents (21, 22) étant agencés à distance l'un de l'autre et définissant deux surfaces de délimitation opposées l'une à l'autre d'un canal d'échantillonnage (25) et l'élément ou les éléments d'étanchéité (26) définissant les parois latérales du canal d'échantillonnage (25), ce qui a pour effet que le canal d'échantillonnage (25) est réalisé sous la forme d'un canal fermé dans la direction longitudinale pourvu d'un orifice d'entrée (20a) et d'un orifice de sortie (20b), au moins un espaceur (23) étant prévu, qui maintient les éléments transparents (21, 22) à distance l'un de l'autre, et au moins l'un des deux éléments transparents (21, 22) présentant une partie en retrait (21a, 22a) qui s'étend en direction de l'autre élément transparent et qui forme une surface de délimitation du canal d'échantillonnage (25), de sorte que la hauteur (h5) du canal d'échantillonnage est inférieure à la hauteur (h6) de l'espaceur ou des espaceurs (23).

8. Hémolysateur comprenant une cuvette interchangeable selon la revendication 7, **caractérisé en ce que** les éléments de production de vibrations (2) peuvent être excités de manière à obtenir des vibrations mécaniques avec leur amplitude de course ajustable.

9. Hémolysateur comprenant une cuvette interchangeable selon la revendication 7, **caractérisé en ce que** la cuvette (20) est intégrée dans un consommable (102) d'un analyseur spectroscopique (100), en particulier dans un pack fluidique, qui comporte des liquides fonctionnels et/ou des récipients à déchets.

10. Hémolysateur comprenant une cuvette interchangeable selon la revendication 7 en combinaison avec un générateur de signaux électriques de courant alternatif (10) pour l'excitation des éléments de production de vibrations (2), **caractérisé en ce que** le générateur de signaux électriques en courant alternatif (10) produit des signaux non sinusoïdaux, par exemple des signaux présentant des formes d'onde pouvant être définies librement et/ou des signaux dont la fréquence peut être accordée.

11. Hémolysateur comprenant une cuvette interchangeable selon la revendication 7 ou 10, **caractérisé en ce que** les éléments de production de vibrations (2) sont serrés entre un premier et un deuxième conducteur (13, 14), où des mats (15) électro-conducteurs sont agencés au niveau des surfaces limites entre l'élément ou les éléments de production de vibrations (2) et les conducteurs, les mats contiennent des particules faites d'un matériau électro-conducteur, en particulier du carbone,.

12. Hémolysateur comprenant une cuvette interchangeable selon la revendication 7, **caractérisé en ce que** les actionneurs multicouches piézoélectriques utilisés comme des capteurs sont conçus de sorte qu'au moyen de leurs signaux, une plage de fréquences optimale et/ou une amplitude de course optimale pour les éléments de production de vibrations de l'hémolysateur en combinaison avec une cuvette peuvent être établies.

13. Analyseur spectroscopique (100) pour l'analyse spectroscopique d'un échantillon (28), tel que par exemple un échantillon complet de sang, situé dans une cuvette (20) au moins en partie transparente, par irradiation de l'échantillon à un faisceau lumineux et détection du spectre du faisceau lumineux après le passage de ce dernier à travers l'échantillon, l'analyseur étant conçu pour réaliser une hémolyse de l'échantillon avant l'iirradiation de l'échantillon à un faisceau lumineux, **caractérisé en ce que** l'analyseur, pour réaliser l'hémolyse, comprend un hémolyseur (1) pourvu d'une cuvette interchangeable selon l'une quelconque des revendications 7 à 12.
